# EUROPÄISCHE PATENTSCHRIFT

(11) **EP 3 302 415 B1**
(45) Veröffentlichungstag und Bekanntmachung des Hinweises auf die Patenterteilung: **24.04.2019**
(21) Anmeldenummer: 16723098.6
(22) Anmeldetag: 13.05.2016
(51) Int. Cl.: A61K 8/38, A61Q 5/08

(54) **MITTEL ZUM BLONDIEREN VON HAAREN MIT ROTER ODER ROT-BLONDER AUSGANGSHAARFARBE**
COMPOSITION FOR LIGHTENING HAIR STARTING WITH RED OR RED-BLONDE COLOR
COMPOSITION POUR LE BLANCHISSEMENT DES CHEVEUX ROUGES OU BLONDS-ROUGES

(30) Priorität: 27.05.2015 DE 102015209739
(43) Veröffentlichungstag der Anmeldung: 11.04.2018
(73) Patentinhaber: Henkel AG & Co. KGaA, 40589 Düsseldorf (DE)
(72) Erfinder: ANDERHEGGEN, Bernd, 41189 Mönchengladbach (DE)
(86) Internationale Anmeldenummer: PCT/EP2016/060832
(87) Internationale Veröffentlichungsnummer: WO 2016/188773

(56) Entgegenhaltungen:
- WO-A1-2014/183881
- WO-A1-2014/183882
- WO-A2-2012/025269
- WO-A2-2014/075861

## Beschreibung

Die vorliegende Erfindung liegt im Bereich der Kosmetik und betrifft Mittel zur Blondierung von keratinischen Fasern, insbesondere menschlichen Haaren. Ein zweiter Gegenstand der Erfindung ist ein Verfahren zur Blondierung von Haaren, insbesondere von Haaren mit roter oder rot-blonder Ausgangshaarfarbe.

Das Blondieren von Haaren beruht auf der oxidativen Zerstörung der in der Haarfaser befindlichen Melanin-Pigmente. Für einen moderaten Blondiereffekt genügt der Einsatz von Wasserstoffperoxid - gegebenenfalls unter Einsatz von Ammoniak oder anderen Alkalisierungsmitteln - als Oxidationsmittel allein. Für das Erzielen eines stärkeren Blondiereffektes wird üblicherweise eine Mischung aus Wasserstoffperoxid und Peroxodisulfatsalzen eingesetzt.

Im Fall einer dunklen Ausganghaarfarbe müssen dabei Eumelanine (schwarze Melanin-Pigmente) und Phäomelanine (rote Melanin-Pigmente), bei hellem oder rötlichem Haar im wesentlichen Phäomelanine (rote Melanin-Pigmente) oxidativ abgebaut werden. Eumelanine und Phäomelanine unterscheiden sich strukturell. Eumelanin entsteht durch enzymatische Oxidation von DOPA, wohingegen Phäomelanine auf biosynthetischem Wege enzymatisch aus 5-S-Cysteinyl-DOPA hervorgehen.

Beide Pigment-Typen zeigen hinsichtlich ihrer Oxidationsstabilität Unterschiede. So sind Eumelanine im Vergleich zu Phäomelaninen gegenüber Oxidationsmitteln empfindlicher und werden durch diese schneller abgebaut. Phäomelanine hingegen besitzen gegenüber Oxidationsmitteln eine höhere Resistenz und werden langsamer bzw. nur unvollständig abgebaut.

Bei der Blondierung von dunklen, beispielsweise dunkelbraunen oder schwarzen Haaren werden daher die Eumelanine im Vergleich zu den Phäomelaninen schneller bzw. in einem stärkeren Ausmaß abgebaut, was oftmals zu einer Verrötung des blondierten Haares führt.

Üblicherweise sind solche Farbverschiebungen in Richtung warmer, rötlicher bis oranger Töne bei dem Anwender höchst unerwünscht. Daher wird dieser Farbverschiebung zumeist durch eine Tönung oder Färbung mit der entsprechenden Komplementärfarbe gemäß der Farbenlehre entgegengewirkt. Ziel ist dabei ein silbrig-kühler Eindruck des Bleichergebnisses. Der Fachmann spricht in diesem Zusammenhang von einer Mattierung.

Zur Vermeidung der Verrötung werden kommerziellen Blondiermitteln somit oft mattierende Farbstoffe zugesetzt. Bei den mattierenden Farbstoffen handelt es sich um blaue oder blau-graue Farbstoffe, die direktziehende Farbstoffe Oxidationsfarbstoffvorprodukte sein können. Die mattierenden Farbstoffe besitzen eine zu den auf den blondierten Haaren verbleibenden Phäomelaninen komplementäre Absorption und können so den roten Farbeindruck kaschieren. Der Einsatz der Mattierungsfarbstoffe ist jedoch auch mit verschiedenen Nachteilen verbunden. So sind die Mattierungsfarbstoffe oft auch selbst gegenüber den Oxidationsmitteln instabil und müssen dann von diesen getrennt konfektioniert werden. Weiterhin können die Mattierungsfarbstoffe im Zuge wiederholter Haarwäschen ausgewaschen werden, so dass die Verrötung mit jeder Haarwäsche wieder stärker wahrnehmbar wird. Schließlich erfolgt durch Verwendung der Mattierfarbstoffe wieder eine Färbung, so dass der gesamte Aufhelleffekt weniger stark ist und ein sehr helles Blond auf diesem Wege nicht erzielt werden kann.

Bei der Blondierung von Haaren mit rötlich-blonder oder roter Ausgangshaarfarbe müssen die für die rote Haarfärbung verantwortlichen Phäomelanine möglichst vollständig oxidativ abgebaut werden. Aufgrund der hohen Resistenz der Phäomelanine gegenüber Oxidationsmitteln ist bei roten bzw. rötlichen Haaren ein signifikanter Aufhelleffekt zur schwer möglich. Gerade bei rotstichigen Haaren führt eine nicht vollständige Blondierung zu einem unattraktiven, hell-rötlichen Farbergebnis, das auch bei Zusatz von mattierenden Farbstoffen kein attraktives Resultat liefert.

Aus dem Stand der Technik sind bislang keine Mittel bekannt, die eine starke Blondierung ohne Verrötung ermöglichen und die bei der Blondierung von roten bzw. rot-blonden Haaren zu einem attraktiven Resultat führen. WO2014/183882 offenbart verschiedene Blondiermittel die Kaliumpersulfat, Ammoniumpersulfat und Natriumperoxodisulfat enthalten.

Aufgabe der vorliegenden Erfindung war es, die Eigenschaften von Blondiermitteln weiter zu verbessern und die zuvor beschriebenen Nachteile zu minimieren. Hierbei sollte insbesondere die bei der Blondierung von dunklen Haaren stattfindende Verrötung minimiert werden. Bei der Blondierung von roten Haaren sollte eine möglichst vollständige Blondierung und ein kompletter Abbau der Phäomelanine ermöglicht werden.

Überraschenderweise hat sich gezeigt, dass Blondiermittel, in denen die drei Peroxodisulfatsalze Kaliumperoxodisulfat, Ammoniumperoxodisulfat und Natriumperoxodisulfat in ganz speziellen Gewichtsverhältnissen zueinander eingesetzt werden, sich bei einer hervorragender Blondierleistung durch eine besonders gute "Rot-Reduktion" auszeichnen, d.h. mit Einsatz dieser Blondiermittel können besonders, klare und helle Blondtöne ohne Rotanteil erzeugt werden.

Ein erster Gegenstand der vorliegenden Erfindung ist ein kosmetisches Mittel zum Aufhellen keratinischer Fasern, enthaltend - bezogen auf sein Gesamtgewicht -
(A) 12,0 bis 23,0 Gew.-% Kaliumperoxodisulfat und
(B) 10,0 bis 20,0 Gew.-% Ammoniumperoxodisulfat und
(C) 5,0 bis 15,0 Gew.-% Natriumperoxodisulfat.

Bei dem Mittel handelt es sich um ein kosmetisches Mittel, d.h. ein Mittel, das für den Einsatz auf dem menschlichen Kopf geeignet ist.

Demnach handelt es sich bei dem ersten Gegenstand der vorliegenden Erfindung um ein kosmetisches Mittel zum Aufhellen menschlicher Haare, enthaltend - bezogen auf sein Gesamtgewicht -
(A) 12,0 bis 23,0 Gew.-% Kaliumperoxodisulfat und
(B) 10,0 bis 20,0 Gew.-% Ammoniumperoxodisulfat und
(C) 5,0 bis 15,0 Gew.-% Natriumperoxodisulfat
dadurch gekennzeichnet, dass - das Gewichtsverhältnis von (A) zu (B)I, d.h. das Gewichtsverhältnis (A)/(B). bei einem Wert von 1.0 bis 1.5 liegt. - das Gewichtsverhältnis von (A) zu (C). d.h. das Gewichtsverhaltnis (A)/(C), bei einem Wert von 1.3 bis 2.1 liegt, und - das Gewichtsverhaltnis von (B) zu (C) d.h. das Gewichtsverhaltnis (B)/(C>. bei einem Wert von 1.1 bis 1.9 liegt.

Unter keratinischen Fasern oder auch Keratinfasern sind dabei Pelze, Wolle, Federn und insbesondere menschliche Haare zu verstehen. Obwohl die erfindungsgemäßen Mittel in erster Linie zum Aufhellen von Keratinfasern geeignet sind, steht prinzipiell einer Verwendung auch auf anderen Gebieten nichts entgegen.

Unter der Aufhellung von keratinischen Fasern wird auch eine Blondierung bzw. eine Bleiche verstanden. Keratinfasern können farbmetrisch vermessen werden (Messung der Lab-Werte). Im farbmetrischen System steht der L-Wert für die Helligkeit einer Farbe bzw. der Keratinfaser (ein L-Wert von 0 bedeutet schwarz, bei einem L-Wert von 100 liegt ein diffuses Weiß vor). Werden aufgehellte Haare farbmetrisch vermessen, so besitzen diese einen höheren L-Wert als vor der Aufhellung.

Erfindungswesentlich für die erfindungsgemäßen Aufhellmittel ist ihr spezieller Gehalt an Kaliumperoxodisulfat, Ammoniumperoxodisulfat und Natriumperoxodisulfat. Wenn alle drei Peroxodisulfate in bestimmten, genau aufeinander abgestimmten Mengenbereichen in den erfindungsgemäßen Mitteln enthalten sind, kann eine besonders starke und klare Aufhellung ohne Verrötung erzielt werden. Auch rote und rot-blonde Keratinfasern können mit diesen Mitteln unter Erhalt eines attraktiven Blondtones aufgehellt werden.

Bei den erfindungsgemäßen Mitteln kann es sich beispielsweise um feste, pulverförmige oder pastenförmige Mittel handeln, die aufgrund ihres Gehaltes an Persulfaten auch als Booster-Pulver, -Gel, oder -Paste bezeichnet werden können. Diese Mittel können beispielsweise als Bestandteil eines Kits (d.h. einer Mehrkomponenten-Verpackungseinheit) vertrieben und kurz vor der Anwendung mit einer zweiten (oder auch mit einer zweiten und einer dritten) Komponente vermischt werden. Bei der zweiten Komponente handelt es sich um eine flüssige, gel- oder cremeförmige Formulierung, welche Wasserstoffperoxid enthält.

Der Verrötungsgrad der aufgehellten Keratinfasern lässt sich durch eine farbmetrische Vermessung der Keratinfasern bestimmen. Im Farbmetrik-System (Lab-Werte) gibt der a-Wert den Rot- bzw. Grünanteil einer Farbe an. Ein negativer a-Wert steht für eine Farbe mit hohem Grünanteil, wohingegen ein positiver a-Wert für eine Farbe mit einem hohen Rotanteil steht. Eine aufgehellte Strähne mit guter Rot-Reduktion besitzt demnach einen möglichst geringen a-Wert.

Das erfindungsgemäße Mittel enthält - bezogen auf sein Gesamtgewicht - 12,0 bis 23,0 Gew.-% Kaliumperoxodisulfat (A). Kaliumperoxodisulfat wird alternativ auch als Kaliumpersulfat bezeichnet und besitzt die Summenformel K₂S₂O₈.

Weiterhin enthält das erfindungsgemäße Mittel - bezogen auf sein Gesamtgewicht - 10,0 bis 20,0 Gew.-% Ammoniumperoxodisulfat (B). Ammoniumperoxodisulfat wird alternativ auch als Ammoniumpersulfat bezeichnet und besitzt die Summenformel (NH₄)₂S₂O₈.

Weiterhin enthält das erfindungsgemäße Mittel - bezogen auf sein Gesamtgewicht - 5,0 bis 15,0 Gew.-% Natriumperoxodisulfat (C). Natriumperoxodisulfat wird alternativ auch als Natriumpersulfat bezeichnet und besitzt die Summenformel Na₂S₂O₈.

Durch Wahl des optimalen Gewichtsverhältnisses von (A)/(B), d.h. von Kaliumperoxodisulfat (A) zu Ammoniumperoxodisulfat (B), kann bei der Blondierung ein Farbresultat mit besonders geringem Rot-Anteil erzielt werden. Hierbei hat sich herausgestellt, dass es zur Minimierung der Verrötung besonders vorteilhaft ist, wenn Kaliumperoxodisulfat in mindestens genau so großer Menge wie Ammoniumperoxodisulfat eingesetzt wird. Besonders bevorzugt wird Kaliumperoxodisulfat in einem leichten, bis 1,5-fachen Gewichtsüberschuss eingesetzt. Daher ist es besonders bevorzugt wenn das Gewichtsverhältnis von Kaliumperoxodisulfat (A) zu Ammoniumperoxodisulfat (B) bei einem Wert von 1,0 bis 1,5, bevorzugt von 1,0 bis 1,4, weiter bevorzugt von 1,0 bis 1,3 und besonders bevorzugt von 1,0 bis 1,2 liegt.

In einer besonders bevorzugten Ausführungsform ist ein erfindungsgemäßes kosmetisches Mittel deshalb dadurch gekennzeichnet, dass das Gewichtsverhältnis von (A) zu (B), d.h. das Gewichtsverhältnis (A)/(B), bei einem Wert von 1,0 bis 1,5, bevorzugt von 1,0 bis 1,4, weiter bevorzugt von 1,0 bis 1,3 und besonders bevorzugt von 1,0 bis 1,2 liegt.

Beispiel: Wenn 100 g eines Booster-Pulvers- neben weiteren kosmetisch akzeptablen Bestandteilen - (A) 17,0 Gew.-% Kaliumperoxodisulfat und (B) 15,0 Gew.-% Ammoniumperoxodisulfat enthalten, dann liegt das Gewichtsverhältnis (A)/(B) bei einem Wert von 1,13.

Auch das Gewichtsverhältnis, in dem Kaliumperoxodisulfat (A) und Natriumperoxodisulfat (C) zueinander eingesetzt werden, nimmt auf die Minimierung der Verrötung Einfluss. Hierbei wurden besonders gute Ergebnisse erhalten, wenn Kaliumperoxodisulfat (A) im Vergleich zu Natriumperoxodisulfat (C) in einem 1,3 bis 2,1-fachen Gewichtsüberschuss eingesetzt wurde.

In einer weiteren besonders bevorzugten Ausführungsform ist ein erfindungsgemäßes kosmetisches Mittel deshalb dadurch gekennzeichnet, dass das Gewichtsverhältnis von (A) zu (C), d.h. das Gewichtsverhältnis (A)/(C), bei einem Wert von 1,3 bis 2,1, bevorzugt von 1,4 bis 2,0, weiter bevorzugt von 1,5 bis 1,9 und besonders bevorzugt von 1,6 bis 1,8 liegt.

Beispiel: Wenn 100 g eines Booster-Pulvers- neben weiteren kosmetisch akzeptablen Bestandteilen - (A) 17,0 Gew.-% Kaliumperoxodisulfat und (C) 10,0 Gew.-% Natriumperoxodisulfat enthalten, dann liegt das Gewichtsverhältnis (A)/(C) bei einem Wert von 1,70.

Schließlich übt auch das Gewichtsverhältnis, in dem Ammoniumperoxodisulfat (B) und Natriumperoxodisulfat (C) zueinander eingesetzt werden, auf den Rot-Anteil der blondierten Keratinfasern einen Einfluss aus. In diesem Zusammenhang hat sich herausgestellt, dass besonders klare Blondtöne dann erzielt werden konnten, denn Ammoniumperoxodisulfat (B) im Vergleich zu Natriumperoxodisulfat (C) ein einem leichten, bevorzugt 1,1 bis 1,9-fachen Gewichtsüberschuss eingesetzt wurde.

In einer weiteren besonders bevorzugten Ausführungsform ist ein erfindungsgemäßes kosmetisches Mittel deshalb dadurch gekennzeichnet, dass das Gewichtsverhältnis von (B) zu (C), d.h. das Gewichtsverhältnis (B)/(C), bei einem Wert von 1,1 bis 1,9, bevorzugt von 1,2 bis 1,8, weiter bevorzugt von 1,3 bis 1,7 und besonders bevorzugt von 1,4 bis 1,6 liegt.

Beispiel: Wenn 100 g eines Booster-Pulvers- neben weiteren kosmetisch akzeptablen Bestandteilen - (B) 15,0 Gew.-% Ammoniumperoxodisulfat und (C) 10,0 Gew.-% Natriumperoxodisulfat enthalten, dann liegt das Gewichtsverhältnis (A)/(C) bei einem Wert von 1,50.

Wie bereits zuvor beschrieben, enthalten die erfindungsgemäßen Mittel - bezogen auf ihr Gesamtgewicht -
(A) 12,0 bis 23,0 Gew.-% Kaliumperoxodisulfat und
(B) 10,0 bis 20,0 Gew.-% Ammoniumperoxodisulfat und
(C) 5,0 bis 15,0 Gew.-% Natriumperoxodisulfat.

Demnach liegt der maximale Gehalt an Persulfaten im erfindungsgemäßen Mittel bei 58 Gew.-%. Ganz besonders bevorzugt ist es, wenn der Gesamtgehalt aller im Mittel enthaltenen Peroxodisulfate - bezogen auf das Gesamtgewicht des Mittels - bei einem Wert von 36,0 bis 48,0 Gew.-%, bevorzugt von 37,0 bis 47,0 Gew.-%, weiter bevorzugt von 38,0 bis 46,0 Gew.-% und besonders bevorzugt von 39,0 bis 45,0 Gew.-% liegt.

Unter dem Gesamtgehalt aller im Mittel enthaltenen Peroxodisulfate wird die Summe aus Kaliumperoxodisulfat (A), Ammoniumperoxodisulfat (B) und Natriumperoxodisulfat (C) verstanden.

In einer weiteren besonders bevorzugten Ausführungsform ist ein erfindungsgemäßes kosmetisches Mittel deshalb dadurch gekennzeichnet, dass der Gesamtgehalt aller im Mittel enthaltenen Peroxodisulfate (A) + (B) + (C) - bezogen auf das Gesamtgewicht des Mittels - bei einem Wert von 36,0 bis 48,0 Gew.-%, bevorzugt von 37,0 bis 47,0 Gew.-%, weiter bevorzugt von 38,0 bis 46,0 Gew.-% und besonders bevorzugt von 39,0 bis 45,0 Gew.-% liegt.

Beispiel: Wenn 100 g eines Booster-Pulvers- neben weiteren kosmetisch akzeptablen Bestandteilen - (A) 17,0 Gew.-% Kaliumperoxodisulfat und (B) 15,0 Gew.-% Ammoniumperoxodisulfat und (C) 10,0 Gew.-% Natriumpersulfat enthalten, dann liegt der Gesamtgehalt an Peroxodisulfaten im Mittel bei 42,0 Gew.-%.

Kaliumperoxodisulfat (A) kann in einer Menge von 12,0 bis 23,0 Gew.-% im erfindungsgemäßen Mittel enthalten sein, wobei die prozentuale Mengenangabe auf das Gesamtgewicht des Mittels bezogen ist. Ganz besonders bevorzugt ist es, wenn Kaliumperoxodisulfat (A) kann in einer Menge von 13,0 bis 22,0 Gew.-%, bevorzugt 14,0 bis 21,0 Gew.-%, weiter bevorzugt 15,0 bis 20,0 Gew.-% und besonders bevorzugt 16,0 bis 19,0 Gew.-% im Aufhellmittel enthalten ist.

In einer weiteren besonders bevorzugten Ausführungsform ist ein erfindungsgemäßes kosmetisches Mittel deshalb dadurch gekennzeichnet, dass es - bezogen auf sein Gesamtgewicht - (A) 13,0 bis 22,0 Gew.-%, bevorzugt 14,0 bis 21,0 Gew.-%, weiter bevorzugt 15,0 bis 20,0 Gew.-% und besonders bevorzugt 16,0 bis 19,0 Gew.-% Kaliumperoxodisulfat enthält.

Ammoniumperoxodisulfat (B) kann in einer Menge von 10,0 bis 20,0 Gew.-% im erfindungsgemäßen Mittel enthalten sein, wobei die prozentuale Mengenangabe auf das Gesamtgewicht des Mittels bezogen ist. Ganz besonders bevorzugt ist es, wenn Ammoniumperoxodisulfat (B) in einer Menge von 11,0 bis 19,0 Gew.-%, bevorzugt 12,0 bis 18,0 Gew.-%, weiter bevorzugt 13,0 bis 17,0 Gew.-% und besonders bevorzugt 14,0 bis 16,0 Gew.-% im Aufhellmittel enthalten ist.

In einer weiteren besonders bevorzugten Ausführungsform ist ein erfindungsgemäßes kosmetisches Mittel deshalb dadurch gekennzeichnet, dass es - bezogen auf sein Gesamtgewicht - (B) 11,0 bis 19,0 Gew.-%, bevorzugt 12,0 bis 18,0 Gew.-%, weiter bevorzugt 13,0 bis 17,0 Gew.-% und besonders bevorzugt 14,0 bis 16,0 Gew.-% Ammoniumperoxodisulfat enthält.

Natriumperoxodisulfat (C) kann in einer Menge von 5,0 bis 15,0 Gew.-% im erfindungsgemäßen Mittel enthalten sein, wobei die prozentuale Mengenangabe auf das Gesamtgewicht des Mittels bezogen ist. Ganz besonders bevorzugt ist es, wenn Natriumperoxodisulfat (C) in einer Menge von 6,0 bis14,0 Gew.-%, bevorzugt 7,0 bis 13,0 Gew.-%, weiter bevorzugt 8,0 bis 12,0 Gew.-% und besonders bevorzugt 9,0 bis 11,0 Gew.-% im Aufhellmittel enthalten ist.

In einer weiteren besonders bevorzugten Ausführungsform ist ein erfindungsgemäßes kosmetisches Mittel deshalb dadurch gekennzeichnet, dass es - bezogen auf sein Gesamtgewicht - (C) 6,0 bis14,0 Gew.-%, bevorzugt 7,0 bis 13,0 Gew.-%, weiter bevorzugt 8,0 bis 12,0 Gew.-% und besonders bevorzugt 9,0 bis 11,0 Gew.-% Natriumperoxodisulfat enthält.

Der maximale Gehalt an Persulfaten im erfindungsgemäßen Mittel liegt bei 58 Gew.-%. Bei den restlichen, mindestens 42 Gew.-% des Mittels handelt es sich um weitere kosmetisch akzeptable Inhaltsstoffe wie beispielsweise Trägerstoffe, Alkalisierungsmittel, Pflegemittel, Verdicker, Tenside, Polymere, Fette, Öle oder ähnliches, die der Fachmann entsprechend den gewünschten Eigenschaften dem Mittel zusetzen kann.

Weiterhin können die erfindungsgemäßen Mittel ein oder mehrere Alkalisierungsmittel enthalten. Geeignete Alkalisierungsmittel sind beispielsweise Ammoniak, Alkanolamine, basischen Aminosäuren, sowie anorganischen Alkalisierungsmittel wie (Erd-)Alkalimetallhydroxide, (Erd-)Alkalimetallmetasilikate, (Erd-) Alkalimetallphosphate und (Erd-)Alkalimetallhydrogenphosphate. Als Metallionen dienen bevorzugt Lithium, Natrium, Kalium und/oder Magnesium.

Erfindungsgemäß einsetzbare, anorganische Alkalisierungsmittel sind bevorzugt ausgewählt aus Natriumhydroxid, Kaliumhydroxid, Magnesiumhydroxid, Calciumhydroxid, Bariumhydroxid, Natriumphosphat, Kaliumphosphat, Natriumsilicat, Kaliumsilicat, Magnesiumsilicat, Natriumcarbonat und Kaliumcarbonat.

Es hat sich als bevorzugt herausgestellt, in den erfindungsgemäßen Zusammensetzungen Metasilikate einzusetzen. Diese steigern die Bleichwirkung bei gleichzeitig verringerter Schädigung der keratinischen Faser. Hier haben sich bevorzugt (Erd-)Alkalimetallmetasilikate, besonders bevorzugt Alkalimetallmetasilikate und insbesondere Natriummetasilikat als geeignet erwiesen. Erfindungsgemäß bevorzugte Mittel enthalten daher - bezogen auf ihr Gewicht - 5 bis < 10 Gew.-%, vorzugsweise 6 bis < 9,5 Gew.-%, weiter bevorzugt 6,5 bis < 9 Gew.-%, besonders bevorzugt 7 bis < 8,5 Gew.-% und insbesondere 7,5 bis < 8 Gew.-% (Erd-)Alkalimetallmetasilikate, bevorzugt Alkalimetallmetasilikate und insbesondere Natriummetasilikat.

Weitere erfindungsgemäß einsetzbare Alkalisierungsmittel können auch ausgewählt werden aus Alkanolaminen aus primären, sekundären oder tertiären Aminen mit einem C₂-C₆-Alkylgrundkörper, der mindestens eine Hydroxylgruppe trägt. Besonders bevorzugte Alkanolamine werden aus der Gruppe ausgewählt, die gebildet wird, aus 2-Aminoethan-1-ol (Monoethanolamin), 3-Aminopropan-1-ol, 4-Aminobutan-1-ol, 5-Aminopentan-1-ol, 1-Aminopropan-2-ol (Monoisopropanolamin), 1-Aminobutan-2-ol, 1-Aminopentan-2-ol, 1-Aminopentan-3-ol, 1-Aminopentan-4-ol, 2-Amino-2-methyl-propanol, 2-Amino-2-methylbutanol, 3-Amino-2-methylpropan-1-ol, 1-Amino-2-methylpropan-2-ol, 3-Aminopropan-1,2-diol, 2-Amino-2-methylpropan-1,3-diol, 2-Amino-2-ethyl-1,3-propandiol, N,N-Dimethyl-ethanolamin, Methylglucamin, Triethanolamin, Diethanolamin und Triisopropanolamin. Insbesondere bevorzugte Alkanolamine sind Monoethanolamin, 2-Amino-2-methyl-propanol und Triethanolamin.

Die als erfindungsgemäßes Alkalisierungsmittel einsetzbaren basischen Aminosäuren werden können ausgewählt werden aus der Gruppe, die gebildet wird aus L-Arginin, D-Arginin, D/L-Arginin, L-Lysin, D-Lysin, D/L-Lysin, L-Ornithin, D- Ornithin, D/L- Ornithin, L-Histidin, D-Histidin und/oder D/L-Histidin.

Ganz besonders bevorzugt ist der Einsatz mindestens eines festen Alkalisierungsmittels aus der Gruppe aus Magnesiumcarbonat, Magnesiumhydrogencarbonat, Magnesiumhydroxid, Calciumcarbonat, Calciumhydrogencarbonat, Calciumhydroxid, Ammoniumcarbonat, Natriumcarbonat, Natriumhydrogencarbonat, Kaliumcarbonat und Kaliumhydrogencarbonat, da der Einsatz dieser Alkalisierungsmittel die Konfektionierung des erfindungsgemäßen Mittels als feste, oder pastöse Booster-Komponente bei gleichzeitiger Verbesserung der Blondierleistung ermöglicht.

In einer weiteren besonders bevorzugten Ausführungsform ist ein erfindungsgemäßes kosmetisches Mittel deshalb dadurch gekennzeichnet, dass es ein oder mehrere Alkalisierungsmittel aus der Gruppe Magnesiumcarbonat, Magnesiumhydrogencarbonat, Magnesiumhydroxid, Calciumcarbonat, Calciumhydrogencarbonat, Calciumhydroxid, Ammoniumcarbonat, Natriumcarbonat, Natriumhydrogencarbonat, Kaliumcarbonat und Kaliumhydrogencarbonat enthält.

Wenn die anwendungsbereiten Mischungen Alkalisierungsmittel enthalten, sind Zubereitungen erfindungsgemäß bevorzugt, die Alkalisierungsmittel in einer Menge von 0,05 bis 20 Gew.-%, insbesondere von 0,5 bis 10 Gew.-%, jeweils bezogen auf das Gesamtgewicht des Mittels, enthalten.

Ganz besonders bevorzugt ist ein Mittel, das -bezogen auf sein Gesamtgewicht - 5,0 bis 20,0 Gew.-%, bevorzugt 6,0 bis 17,5 Gew.-%, weiter bevorzugt 7,0 bis 15,0 Gew.-% und besonders bevorzugt 12,5 Gew.-% Magnesiumcarbonat enthält.

In einer weiteren besonders bevorzugten Ausführungsform ist ein erfindungsgemäßes kosmetisches Mittel deshalb dadurch gekennzeichnet, dass es - bezogen auf sein Gesamtgewicht - 5,0 bis 20,0 Gew.-%, bevorzugt 6,0 bis 17,5 Gew.-%, weiter bevorzugt 7,0 bis 15,0 Gew.-% und besonders bevorzugt 9,0 bis 12,5 Gew.-% Magnesiumcarbonat enthält.

Zusätzlich können die erfindungsgemäßen Aufhellmittel auch noch weitere Inhaltsstoffe enthalten.

Als Konsistenzgeber können die erfindungsgemäßen Mittel zusätzlich ein oder mehrere Fettalkohole enthalten, die 6 bis 30 Kohlenstoffatome in ihrer Alkylkette tragen. Dabei kann die Alkylkette sowohl eine oder mehrere Verzweigungen als auch cis- und/oder trans-konfigurierte Doppelbindungen enthalten. Beispiele sind hierfür Hexylalkohol (Capronalkohol), Heptylalkohol (Önanthalkohol), Octylalkohol (Caprylalkohol), Nonylalkohol (Pelargonylalkohol), Undecylalkohol, Undec-10-en-1-ol, Dodecylalkohol (Laurylalkohol), 2,6,8-Trimethyl-4-nonanol (Isolaurylalkohol), Tridecylalkohol, Tetradecylalkohol (Myristylalkohol), Pentadecylalkohol, Hexadecylalkohol (Cetylalkohol, oder Palmitylalkohol), Heptadecylalkohol, Octadecylalkohol (Stearylalkohol), Isostearylalkohol, (9*Z*)-Octadec-9-en-1-ol (Oleylalkohol), (9*E*)-Octadec-9-en-1-ol (Elaidylalkohol), (9*Z*,12*Z*)-Octadeca-9,12-dien-1-ol (Linoleylalkohol), (9*Z*,12*Z*,15*Z*)-Octadeca-9,12,15-trien-1-ol (Linolenylalkohol), Nonadecan-1-ol (Nonadecylalkohol), Eicosan-1-ol (Eicosylalkohol / Arachylalkohol), (9*Z*)-Eicos-9-en-1-ol (Gadoleylalkohol), (5*Z*,8*Z*,11*Z*,14*Z*)-Eicosa-5,8,11,14-tetraen-1-ol (Arachidonalkohol), Heneicosylalkohol, Docosylalkohol (Behenylalkohol), (13*Z*)-Docos-13-en-1-ol (Erucylalkohol) oder (13*E*)-Docosen-1-ol (Brassidylalkohol). Es ist erfindungsgemäß ebenfalls möglich, Gemische von Fettalkoholen, die durch gezielte Mischung oder auch durch Gewinnungsverfahren als solche anfallen, einzusetzen. Beispiele sind Cocosalkohol (Mischung aus C₈-C₁₈-Fettalkoholen) oder Cetearylalkohol (1:1-Mischung aus C₁₆- und C₁₈-Fettalkoholen).

Weiterhin geeignete nichtionische Inhaltsstoffe sind Ethylenglykolether von Fettalkoholen. Prinzipiell sind dabei Ethylenglykolether der bereits genannten Fettalkohole einsetzbar. Geeignet sind beispielsweise Ethylenglykolether mit einem Ethoxylierungsgrad von 1 (Fettalkoholmonoethylenglykolether), 2 (Fettalkoholdiethylenglykolether) oder 3 (Fettalkoholtriethylenglykolether). Erfindungsgemäß bevorzugte Ethylenglykolether sind beispielsweise Laureth-1, Laureth-2, Laureth-3, Isolaureth-1, Isolaureth-2, lsolaureth-3, Trideceth-1, Trideceth-2, Trideceth-3, Myreth-1, Myreth-2, Myreth-3, Ceteth-1, Ceteth-2, Ceteth-3, Steareth-1, Steareth-2, Steareth-3, Oleth-1, Oleth-2, Oleth-3, Ceteareth-1, Ceteareth-2, Ceteareth-3, Coceth-1, Coceth-2, Cocoeth-3, Pareth-1, Pareth-2 und Pareth-3. Insbesondere bevorzugt sind Ceteth-1, Laureth-2, Ceteth-2, Steareth-2, Oleth-2, Laureth-3, Isolaureth-3, Trideceth-3, Ceteareth-3 und Oleth-3.

Weiterhin geeignete Inhaltsstoffe sind Anlagerungsprodukte von Fettalkoholen mit Propylenoxid. Prinzipiell sind dabei Propylenglykolether der bereits genannten Fettalkohole einsetzbar, wobei Propylenglykolether mit Fettalkoholen mit niedrigem Propylenierungsgrad bevorzugt sind. Unter Propylenierungsgrad wird dabei die Molmenge an Propylenoxid verstanden, die pro Mol Fettalkohol eingesetzt wurde. Bevorzugt sind Propylenglykolether mit einem Propylenierungsgrad von 1 bis 3. Erfindungsgemäß bevorzugte Ethylenglykolether sind beispielsweise PPG-1 Laurylether, PPG-2 Laurylether, PPG-3 Laurylether, PPG-1 Isolaurylether, PPG-2 Isolaurylether, PPG-3 Isolaurylether, PPG-1 Tridecylether, PPG-2 Tridecylether, PPG-3 Tridecylether, PPG-2 Myristylether, PPG-3 Myristylether, PPG-1 Cetylether, PPG-2 Cetylether, PPG-3 Cetylether, PPG-1 Stearylether, PPG-2 Stearylether, PPG-3 Stearylether, PPG-1 Oleylether, PPG-2 Oleylether, PPG-3 Oleylether, PPG-1 Cetearylether, PPG-2 Cetearylether, PPG-3 Cetearylether, PPG-1 Cocoylether, PPG-2 Cocoylether und PPG-3 Cocoylether. Insbesondere bevorzugt ist PPG-3 Myristylether.

Geeignete Emulgatoren sind beispielsweise Fettsäureester mehrwertiger Alkohole mit gesättigten oder ungesättigten Fettsäuren mit 8 bis 22, insbesondere 10 bis 18, Kohlenstoffatomen in der Fettsäuregruppe einsetzbar. Als mehrwertige Alkohole sind dabei Ethylenglykol, Propylenglykol, Glycerin, Pentaerythrit, Sorbitan oder Zucker sowie Homo- oder Heterooligomere davon bevorzugt.

Erfindungsgemäße Ester von Ethylenglykol oder Propylenglykol sind dabei sowohl die Monofettsäureester wie auch die Difettsäureester mit (Poly-)ethylen- und/oder -propylenglykolen. Bevorzugte Fettsäuren sind beispielsweise Laurin-, Myristin-, Palmitin-, Stearin-, Isostearin- und Ölsäure. Bevorzugte Verbindungen umfassen dabei Ethylenglykolmonofettsäureester, Propylenglykolmonofettsäureester, Ethylenglykoldifettsäureester, Polyethylenglykolmonofettsäureester und Polyethylenglykoldifettsäureester. Erfindungsgemäß besonders bevorzugte Verbindungen sind zum Beispiel Ethylenglykoldistearat, Ethylenglykolmonostearat, Propylenglykolmonostearat, Diethylenglykolmonostearat, Polyethylenglykol(100)monostearat, Polyethylenglykol(200)monostearat, Diethylenglykolmonolaurat, Polyethylenglykol(200)dilaurat, Polyethylenglykol(100)monolaurat, Polyethylenglykol(100)monooleat, Polyethylenglykol(200)dioleat oder Polyethylenglykol(400)dioleat.

Ebenso geeignet sind Mono-, Di- oder Trifettsäureester an Glycerin, dessen Oligomere oder dessen Anlagerungsprodukte mit Ethylenoxid und/oder Propylenoxid. Insbesondere Glycerinmonofettsäureester, Glycerindifettsäureester und Glycerintrifettsäureester sind bevorzugt. Beispielhafte Verbindungen dieses Typs sind Glyceryltrilaurat, Glyceryltrimyristat, Glyceryltripalmitat, Glyceryltristearat, Glyceryltriisostearat, Glyceryltrioleat, Glyceryltricocoat, Glyceryldilaurat, Glyceryldimyristat, Glyceryldipalmitat, Glyceryldistearat, Glyceryldiisostearat, Glyceryldioleat, Glyceryldicocoat, Glycerylmonolaurat, Glycerylmonomyristat, Glycerylmonopalmitat, Glycerylmonostearat, Glycerylmonoisostearat, Glycerylmonooleat und Glycerylmonococoat. Weiterhin sind auch Mono- oder Polyfettsäureester an Polyglyceride erfindungsgemäß einsetzbar. Hierzu sind beispielhaft Decaglycerindecaoleat, Decaglycerinoctaoleat, Decaglcerindecastearat, Trigylceryldiisostearat oder Diglycerinmonostearat genannt.

Ebenso ist es möglich, Triglyceride von Hydroxygruppen-tragenden Fettsäuren einzusetzen, insbesondere Ricinusöl, welches gehärtet oder ungehärtet verwendet werden kann. Ethoxyliertes Ricinusöl ist besonders bevorzugt.

Ebenfalls geeignete, erfindungsgemäße Inhaltsstoffe sind Fettsäureester an Pentaerythrit, insbesondere Pentaerythritmonofettsäureester. Beispiele dieser Verbindungen sind Pentaerythritmonolaurat, Pentaerythritmonomyristat, Pentaerythritmonopalmitat, Pentaerythritmonostearat und Pentaerythritmonooleat.

Weiterhin können die erfindungsgemäßen Mittel auch ein oder mehrere Silikonöle enthalten, die beispielsweise aus der Gruppe der cyclische Siliconöle mit der INCI-Bezeichnung Cyclomethicone ausgewählt werden können. Unter der INCI-Bezeichnung Cyclomethicone werden insbesondere Cyclotrisiloxan (Hexamethylcyclotrisiloxan), Cyclotetrasiloxan (Octamethylcyclotetrasiloxan), Cyclopentasiloxan (Decamethylcyclopentasiloxan) und Cyclohexasiloxan (Dodecamethylcyclohexasiloxan) verstanden.

Ein erfindungsgemäß geeigneter Cyclomethicone-Ersatzstoff ist eine Mischung aus C₁₃-C₁₆-Isoparaffinen, C₁₂ - C₁₄-lsoparaffinen und C₁₃ - C₁₅-Alkanen.

Weitere geeignete Inhaltsstoffe sind auch C₈-C₁₆-lsoparaffine, insbesondere aus Isononan, Isodecan, Isoundecan, Isododecan, Isotridecan, Isotetradecan, Isopentadecan und Isohexadecan, sowie Mischungen hiervon.

Die vorgenannten Inhaltsstoffe können im Mittel jeweils in einer Menge von 0,1 bis 20,0 Gew-%, bezogen auf das Gesamtgewicht des Mittels, enthalten sein.

Von den vorgenannten Inhaltsstoffen haben sich einige als besonders geeignet erwiesen, da sie die physikalische und chemische Stabilität von Blondiermitteln - sofern sie in Pastenform konfektioniert werden - über lange Zeiträume garantieren und mit den weiteren erfindungsgemäßen Inhaltsstoffen hervorragend verträglich sind. Erfindungsgemäß bevorzugte Mittel sind dadurch gekennzeichnet, dass sie mindestens einen hydrophoben Inhaltsstoff aus der Gruppe Paraffinöl, Polyisobuten, der Alkylbenzoate, Isopropylpalmitat, Isohexadekan, Isododecan, Isononyl-Isononanoat enthalten.

Als weiteren Inhaltsstoff können die erfindungsgemäßen Mittel auch ein oder mehrere Polymere enthalten. Geeignet sind beispielsweise Polymere oder Copolymere von Ethylen/Propylen/Styrol und/oder der Copolymere von Butylen/Ethylen/Styrol und/oder der Copolymere von Butylen/Propylen/Styrol. Die Mittel können auch mindestens ein natürliches Polymer enthalten. Als natürliches Polymer können beispielsweise Cellulosederivate verwendet werden, die als Verdickungsmittel Verwendung finden. Beispiele sind Agar-Agar, Carrageen, Alginate, Xanthan-Gum, Karaya-Gummi, Ghatti-Gummi, Tragant, Skleroglucangums oder Gummi arabicum, Alginate, Pektine, Polyosen, Guar-Gums, Johannisbrotbaumkernmehl, Leinsamengummen, Dextrane, Pektine, Staerke-Fraktionen und Derivate wie Amylose, Amylopektin und Dextrine, Gelatine und Casein sowie Cellulosederivate, wie beispielsweise Methylcellulose, Carboxyalkylcellulosen wie Carboxymethylcellulose und Hydroxyalkylcellulosen wie Hydroxyethylcellulose.

Natürliche Polymere aus den genannten Substanzklassen sind kommerziell erhältlich und werden beispielsweise unter den Handelsnamen Deuteron®-XG (anionisches Heteropolysaccharid auf Basis von β-D-Glucose, D-Manose, D-Glucuronsaeure, Schoener GmbH), Deuteron®-XN (nichtionogenes Polysaccharid, Schoener GmbH), Protanal RF 6650 alginate (Natriumalginat, FMC Biopolymer), Cekol (Cellulose Gum, Kelco), Kelzan (Xanthan-Biopolymer, Kelco), Xanthan FN (Xanthan-Biopolymer, Jungbunzlauer), Keltrol z.B. Keltrol CG-T (Xanthan-Biopolymer, Kelco) oder Keltrol CG-SFT (Xanthan-Biopolymer, Kelco) angeboten.

In einer weiteren Ausführungsform der Erfindung enthalten die erfindungsgemäßen Mittel Xanthan. Erfindungsgemäß bevorzugt sind solche Xanthane, die nach der Quellung transparente Zubereitungen ergeben. Insbesondere bevorzugt ist die Verwendung des Xanthan-Biopolymers, welches unter dem Handelsnamen Keltrol CG-SFT der Firma Kelco vertrieben wird.

In einer bevorzugten Ausführungsform enthält ein erfindungsgemäßes Mittel 0,1 bis 5 Gew.-%, vorzugsweise 0,5 bis 4 Gew.-%, weiter bevorzugt 1 bis 3 Gew.-%, besonders bevorzugt 1,25 bis 2,5 Gew.-% und insbesondere 1,5 bis 2 Gew.-% Xanthan.

Die erfindungsgemäßen Zusammensetzungen können zusätzlich mindestens einen weiteren Bleichverstärker, der von den anorganischen Persalzen verschieden ist, enthalten.

Als Bleichverstärker können Verbindungen, die unter Perhydrolysebedingungen aliphatische Peroxocarbonsäuren mit vorzugsweise 1 bis 10 C-Atomen, insbesondere 2 bis 4 C-Atomen, und/oder gegebenenfalls substituierte Perbenzoesäure ergeben, eingesetzt werden. Geeignet sind Substanzen, die O- und/oder N-Acylgruppen der genannten C-Atomzahl und/oder gegebenenfalls substituierte Benzoylgruppen tragen. Bevorzugt sind mehrfach acylierte Alkylendiamine, insbesondere Tetraacetylethylendiamin (TAED), acylierte Triazinderivate, insbesondere 1,5-Diacetyl-2,4-dioxohexahydro-1,3,5-triazin (DADHT), acylierte Glykolurile, insbesondere Tetraacetylglykoluril (TAGU), N-Acylimide, insbesondere N-Nonanoylsuccinimid (NOSI), acylierte Phenolsulfonate, insbesondere n-Nonanoyl- oder Isononanoyloxybenzolsulfonat (n- bzw. iso-NOBS), Carbonsäureanhydride, insbesondere Phthalsäureanhydrid, acylierte mehrwertige Alkohole, insbesondere Triacetin, Ethylenglykoldiacetat und 2,5-Diacetoxy-2,5-dihydrofuran.

Bei den zuvor beschriebenen erfindungsgemäßen Mitteln handelt es sich um persulfathaltige Booster-Komponenten, die in Verfahren zum Aufhellen, insbesondere zum Bleichen von Keratinfasern verwendet werden können.

In optimaler Weise wird das erfindungsgemäße Mittel dem Anwender oder Friseur in Form eines Kits (einer Mehrkomponenten-Verpackungseinheit) zur Verfügung gestellt, welche neben der die Peroxodisulfate enthaltenden ersten Komponente (K1) mindestens eine weitere Komponente (K2) enthält. Bei der zweiten Komponente (K2) handelt es sich um eine Oxidationsmittelzubereitung mit Wasserstoffperoxid. Die Oxidationsmittelzubereitung (K2) liegt bevorzugt in flüssiger, cremeförmiger oder gelförmiger Form vor. Kurz vor der Anwendung werden die beiden Komponenten (K1) und (K2) miteinander vermischt und auf diese Weise das anwendungsbereite Aufhellmittel hergestellt. Ebenfalls möglich und erfindungsgemäß ist es, wenn zur Herstellung des anwendungsbereiten Aufhellmittels die Komponenten (K1), (K2) und zusätzlich (K3) miteinander vermischt werden, wobei es sich bei der Komponente (K3) um eine Komponente handeln kann, welche beispielsweise eine pflegende Substanz, ein spezielles Alkalisierungsmittel oder einen oder mehrere andere, mit den Komponenten (K1) und (K2) nicht kompatible Inhaltsstoffe enthält.

Ein zweiter Gegenstand der vorliegenden Erfindung ist ein Verfahren zur Aufhellung keratinischer Fasern, umfassend die folgenden Schritte in der angegebenen Reihenfolge
(i) Herstellung eines anwendungsbereiten Mittels zur Aufhellung keratinischer Fasern durch Vermischen einer ersten Komponente (K1) mit einer zweiten Komponente (K2),
(ii) Verteilung des anwendungsbereiten Mittels auf den keratinischen Fasern,
(iii) Verbleib des Mittels für einen Zeitraum von 1 bis 60 Minuten auf den Fasern und
(iv) Auswaschen des Mittels aus den Fasern,
wobei
- die erste Komponente (K1) ein Mittel des ersten Erfindungsgegenstands ist und
- die zweite Komponente (K2) eine Oxidationsmittelzubereitung ist, die Wasserstoffperoxid enthält.

Die anwendungsbereiten Mittel werden unmittelbar vor der Anwendung auf dem Haar durch Mischen der zwei Zubereitungen (K1) und (K2) und gegebenenfalls einer dritten Zubereitung (K3) und/oder weiteren Zubereitungen hergestellt. Bei anwendungsbereiten Mitteln, die aus mehr als zwei Zubereitungen zu einer fertigen Anwendungsmischung vermischt werden, kann es unerheblich sein, ob zunächst zwei Zubereitungen miteinander vermischt werden und anschließend die dritte Zubereitung zugegeben und untergemischt wird, oder ob alle Zubereitungen gemeinsam zusammengeführt und anschließend vermischt werden. Das Vermischen kann durch Verrühren in einer Schale oder einem Becher erfolgen oder durch Schütteln in einem verschließbaren Behälter.

Die Schritte (i) und (ii) werden hierbei unmittelbar nacheinander durchgeführt. Der Begriff "unmittelbar" ist dabei als Zeitraum von wenigen Sekunden bis eine Stunde, vorzugsweise bis 30 min, insbesondere bis 15 min zu verstehen.

Die erfindungsgemäßen Mittel werden in einem Verfahren zum Aufhellen von keratinischen Fasern, insbesondere menschlichen Haaren, angewendet, bei dem das Mittel auf die keratinhaltigen Fasern aufgebracht, bei einer Temperatur von Raumtemperatur bis 45 °C für eine Einwirkdauer von 1 bis 60 Minuten auf der Faser belassen und anschließend mit Wasser wieder ausgespült oder mit einem Shampoo ausgewaschen wird.

Die Einwirkzeit der anwendungsbereiten Aufhellmittel kann 1 bis 60 min betragen und liegt bevorzugt bei 15 bis 45 min, besonders bevorzugt bei 20 bis 40 min. Während der Einwirkzeit des Mittels auf der Faser kann es vorteilhaft sein, den Aufhellvorgang durch Wärmezufuhr zu unterstützen. Die Wärmezufuhr kann durch eine externe Wärmequelle, wie mit Hilfe eines Warmluftgebläses, als auch, insbesondere bei einer Haaraufhellung am lebenden Probanden, durch die Körpertemperatur des Probanden erfolgen. Bei letzterer Möglichkeit wird üblicherweise die aufzuhellende Partie mit einer Haube abgedeckt. Eine Einwirkphase bei Raumtemperatur ist ebenfalls erfindungsgemäß. Bevorzugt liegt die Temperatur während der Einwirkzeit zwischen 20°C und 40°C, insbesondere zwischen 25°C und 38°C. Die Aufhellmittel ergeben insbesondere bei physiologisch verträglichen Temperaturen von unter 40°C gute Blondier- und Aufhellergebnisse.

Nach Ende der Einwirkzeit wird die Aufhellzubereitung mit Wasser oder einem Reinigungsmittel aus dem Haar gespült. Als Reinigungsmittel kann dabei insbesondere handelsübliches Shampoo dienen, wobei insbesondere dann auf das Reinigungsmittel verzichtet werden kann und der Ausspülvorgang mit Leitungswasser erfolgen kann, wenn das Aufhellmittel einen stark tensid-haltigen Träger besitzt.

Die anwendungsbereiten Aufhellmittel enthalten sowohl die zuvor beschriebenen Peroxodisulfate in ihren bestimmten Mengenverhältnissen als auch Wasserstoffperoxid und stellen eine reaktive Mischung dar, welche die Eumelanine und insbesondere auch die Phäomelanine des Haares effektiv oxidiativ zerstört.

Um ein anwendungsbereites Aufhellmittel mit optimaler Zusammensetzung zu erhalten, werden die Komponente (K1) und die Komponente (K2) daher bevorzugt in einem bestimmten Mischungsverhältnis miteinander vermischt. Bevorzugt werden die Komponenten (K1) und (K2) dabei in einem Mischungsverhältnis (K1)/(K2) von 0,3 bis 1,0, bevorzugt von 0,3 bis 0,9, weiter bevorzugt von 0,3 bis 0,8 und besonders bevorzugt von 0,3 bis 0,7 miteinander vermengt. Ein Mischungsverhältnis von (K1)/(K2) von 1,0 bedeutet, dass ein Gewichtsanteil der die Peroxodisulfate enthaltenden Komponente (K1) und ein Gewichtsanteil der Wasserstoffperoxid-haltigen Zubereitung (K2) vermischt werden. Analog bedeutet ein Mischungsverhältnis von (K1)/(K2) von 0,5, dass ein Gewichtsanteil der die Peroxodisulfate enthaltenden Komponente (K1) und zwei Gewichtsanteile der Wasserstoffperoxid-haltigen Zubereitung (K2) miteinander vermischt werden.

Ein ganz besonders bevorzugtes Verfahren ist dadurch gekennzeichnet, dass die erste Komponente (K1) und die zweite Komponente (K2) in einem Gewichtsverhältnis (K1)/(K2) von 0,3 bis 1,0, bevorzugt von 0,3 bis 0,9, weiter bevorzugt von 0,3 bis 0,8 und besonders bevorzugt von 0,3 bis 0,7 miteinander vermischt werden.

Bei der Komponente (K2) handelt es sich um die Oxidationsmittelzubereitung, welche Wasserstoffperoxid enthält. In einer bevorzugten Ausführungsform wird Wasserstoffperoxid selbst als wässrige Lösung in der Oxidationsmittelzubereitung (K2) verwendet. Die Konzentration einer Wasserstoffperoxid-Lösung in der Färbezubereitung (K2) wird einerseits von den gesetzlichen Vorgaben und andererseits von dem gewünschten Effekt bestimmt; vorzugsweise werden 1 bis 12 Gew.-%ige Lösungen in Wasser verwendet.

Erfindungsgemäß bevorzugte Zubereitungen (K2) sind dadurch gekennzeichnet, dass sie - bezogen auf das Gesamtgewicht der Komponente (K2) - 1,5 bis 12,0 Gew.-%, bevorzugt 3,0 bis 11,0 Gew.-%, weiter bevorzugt 4,5 bis 10,0 Gew.-% und besonders bevorzugt 5,5 bis 9,5 Gew.-% Wasserstoffperoxid enthalten.

Ein ganz besonders bevorzugtes Verfahren ist dadurch gekennzeichnet, dass die Komponente (K2) - bezogen auf das Gesamtgewicht der Komponente (K2) - 1,5 bis 12,0 Gew.-%, bevorzugt 3,0 bis 11,0 Gew.-%, weiter bevorzugt 4,5 bis 10,0 Gew.-% und besonders bevorzugt 5,5 bis 9,5 Gew.-% Wasserstoffperoxid enthält.

Die Komponente (K2) enthält das Wasserstoffperoxid üblicherweise in einem kosmetischen Träger, der bevorzugt wässrig, alkoholisch oder wässrig-alkoholisch ist. Zum Zwecke der Haarbehandlung sind solche Träger beispielsweise Cremes, Emulsionen, Gele oder auch tensidhaltige schäumende Lösungen, wie beispielsweise Shampoos, Schaumaerosole oder andere Zubereitungen, die für die Anwendung auf dem Haar geeignet sind.

Die die Perooxodisulfate enthaltende Komponente (K1) und/oder die Oxidationsmittelzubereitung (K2) können zur Verstärkung der aufhellenden Wirkung weitere Bleichkraftverstärker enthalten, wie beispielsweise Tetraacetylethylendiamin (TAED), 1,5-Diacetyl-2,4-dioxohexahydro-1,3,5-triazin (DADHT), Tetraacetylglykoluril (TAGU), N-Nonanoylsuccinimid (NOSI), n-Nonanoyl- oder Isononanoyloxybenzolsulfonat (n- bzw. i-NOBS), Phthalsäureanhydrid, Triacetin, Ethylenglykoldiacetat und 2,5-Diacetoxy-2,5-dihydrofuran sowie Carbonatsalze bzw. Hydrogencarbonatsalze, insbesondere Ammoniumhydrogencarbonat, Ammoniumcarbonat, Natriumhydrogencarbonat, Dinatriumcarbonat, Kaliumhydrogencarbonat, Dikaliumcarbonat und Calciumcarbonat, und stickstoffhaltige, heterocyclische Bleichkraftverstärker, wie 4-Acetyl-1-methylpyridinium-p-toluolsulfonat, 2-Acetyl-1-methylpyridinium-p-toluolsulfonat, sowie N-Methyl-3,4-dihydroisochinolinium-p-toluolsulfonat.

Zur weiteren Steigerung der Aufhellung können der Komponente (K1) und/oder der Komponente (K2) zusätzlich mindestens eine SiO₂-Verbindung, wie Kieselsäure oder Silicate, insbesondere Wassergläser, zugesetzt sein. Die SiO2-Verbindung kann hierbei in der Färbezubereitung (K1) und/oder in der Oxidationsmittelzubereitung (K2) enthalten sein. Es kann erfindungsgemäß bevorzugt sein, die SiO₂-Verbindungen in Mengen von 0,05 Gew.-% bis 15 Gew.-%, besonders bevorzugt in Mengen von 0,15 Gew.-% bis 10 Gew.-% und ganz besonders bevorzugt in Mengen von 0,2 Gew.-% bis 5 Gew.-%, jeweils bezogen auf das Gesamtgewicht der Färbezubereitung (K1) bzw. auf das Gesamtgewicht der Oxidationsmittelzubereitung (K2), einzusetzen. Die Mengenangaben geben dabei jeweils den Gehalt der SiO₂-Verbindungen (ohne deren Wasseranteil) in den Mitteln wieder.

Die Komponenten (K1) und/oder (K2) können weiterhin zusätzliche Wirk-, Hilfs- und Zusatzstoffe enthalten, um die Färbe- bzw. Aufhellleistung zu verbessern und weitere gewünschte Eigenschaften der Mittel einzustellen.

Vorzugsweise werden die anwendungsbereiten Aufhellmittel als flüssige Zubereitung bereitgestellt und den Mitteln daher gegebenenfalls zusätzlich eine weitere oberflächenaktive Substanz zugesetzt, wobei solche oberflächenaktive Substanzen je nach Anwendungsgebiet als Tenside oder als Emulgatoren bezeichnet werden: Sie sind bevorzugt aus anionischen, zwitterionischen, amphoteren und nichtionischen Tensiden und Emulgatoren ausgewählt.

Erfindungsgemäß geeignete anwendungsbereite Mittel sind dadurch gekennzeichnet, dass das Mittel (d.h. die Komponente (K1) und/oder (K2)) zusätzlich mindestens ein anionisches Tensid enthält. Geeignete anionische Tenside sind Fettsäuren, Alkylsulfate, Alkylethersulfate und Ethercarbonsäuren mit 10 bis 20 C-Atomen in der Alkylgruppe und bis zu 16 Glykolethergruppen im Molekül.

Erfindungsgemäß geeignete Mittel sind dadurch gekennzeichnet, dass das Mittel (d.h. die Komponente (K1) und/oder (K2)) zusätzlich mindestens ein zwitterionisches Tensid enthält. Bevorzugte zwitterionische Tenside sind Betaine, N-Alkyl-N,N-dimethylammonium-glycinate, N-Acyl-aminopropyl-N,N-dimethylammoniumglycinate, und 2-Alkyl-3-carboxymethyl-3-hydroxyethyl-imidazoline. Ein bevorzugtes zwitterionisches Tensid ist unter der INCI-Bezeichnung Cocamidopropyl Betaine bekannt.

Erfindungsgemäß geeignete Mittel sind weiterhin dadurch gekennzeichnet, dass das Mittel (d.h. die Komponente (K1) und/oder (K2)) zusätzlich mindestens ein amphoteres Tensid enthält. Bevorzugte amphotere Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren. Besonders bevorzugte amphotere Tenside sind N-Kokosalkylaminopropionat, as Kokosacylaminoethylaminopropionat und C₁₂-C₁₈-Acylsarcosin.

Weiterhin hat es sich als vorteilhaft erwiesen, wenn die Komponente (K2) weitere, nichtionogene grenzflächenaktive Stoffe, enthalten. Bevorzugte nichtionische Tenside sind Alkylpolyglykoside sowie Alkylenoxid-Anlagerungsprodukte an Fettalkohole und Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten.

Die nichtionischen, zwitterionischen oder amphoteren Tenside werden in Anteilen von 0,1 bis 45 Gew.%, bevorzugt 1 bis 30 Gew.% und ganz besonders bevorzugt von 1 bis 15 Gew.%, bezogen auf die Gesamtmenge der Komponente (K2), eingesetzt.

Die Komponenten (K1) und/oder (K2) können auch mindestens ein Verdickungsmittel enthalten. Bezüglich dieser Verdickungsmittel bestehen keine prinzipiellen Einschränkungen. Es können sowohl organische als auch rein anorganische Verdickungsmittel zum Einsatz kommen.

Geeignete Verdickungsmittel sind anionische, synthetische Polymere, kationische, synthetische Polymere, natürlich vorkommende Verdickungsmittel, wie nichtionische Guargums, Skleroglucangums oder Xanthangums, Gummi arabicum, Ghatti-Gummi, Karaya-Gummi, Tragant-Gummi, Carrageen-Gummi, Agar-Agar, Johannisbrotkernmehl, Pektine, Alginate, Stärke-Fraktionen und Derivate wie Amylose, Amylopektin und Dextrine, sowie Cellulosederivate, wie beispielsweise Methylcellulose, Carboxyalkylcellulosen und Hydroxyalkylcellulosen, nichtionische, vollsynthetische Polymere, wie Polyvinylalkohol oder Polyvinylpyrrolidinon; sowie anorganische Verdickungsmittel, insbesondere Schichtsilikate wie beispielsweise Bentonit, besonders Smektite, wie Montmorillonit oder Hectorit.

Weiterhin hat es sich als vorteilhaft erweisen, wenn die Aufhellmittel (d.h. die Komponente (K1) und/oder (K2)), insbesondere wenn sie zusätzlich Wasserstoffperoxid enthalten, mindestens einen Stabilisator oder Komplexbildner enthalten. Besonders bevorzugte Stabilisatoren sind Phenacetin, Alkalibenzoate (Natriumbenzoat) und Salicylsäure. Weiterhin können alle Komplexbildner des Standes der Technik eingesetzt werden. Erfindungsgemäß bevorzugte Komplexbildner sind stickstoffhaltigen Polycarbonsäuren, insbesondere EDTA und EDDS, und Phosphonate, insbesondere 1-Hydroxyethan-1,1-diphosphonat (HEDP) und/oder Ethylendiamintetramethylenphosphonat (EDTMP) und/oder Diethylentriaminpentamethylenphosphonat (DTPMP) bzw. deren Natriumsalze.

Ferner können die erfindungsgemäßen Mittel weitere Wirk-, Hilfs- und Zusatzstoffe enthalten, wie beispielsweise nichtionische Polymere wie beispielsweise Vinylpyrrolidinon/Vinylacrylat-Copolymere, Polyvinylpyrrolidinon, Vinylpyrrolidinon/Vinylacetat-Copolymere, Polyethylenglykole und Polysiloxane; zusätzliche Silikone wie flüchtige oder nicht flüchtige, geradkettige, verzweigte oder cyclische, vernetzte oder nicht vernetzte Polyalkylsiloxane (wie Dimethicone oder Cyclomethicone), Polyarylsiloxane und/oder Polyalkylarylsiloxane, insbesondere Polysiloxane mit organofunktionelle Gruppen, wie substituierten oder unsubstituierten Aminen (Amodimethicone), Carboxyl-, Alkoxy- und/oder Hydroxylgruppen (Dimethiconcopolyole), lineare Polysiloxan(A)-Polyoxyalkylen(B)-Blockcopolymere, gepfropften Silikonpolymere; kationische Polymere wie quaternisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quaternierte Dimethylamino-ethylmethacrylat-Vinylpyrrolidinon-Copolymere, Vinylpyrrolidinon-Imidazolinium-methochlorid-Copolymere und quaternierter Polyvinylalkohol; zwitterionische und amphotere Polymere; anionische Polymere wie beispielsweise Polyacrylsäuren oder vernetzte Polyacrylsäuren; Strukturanten wie Glucose, Maleinsäure und Milchsäure, haarkonditionierende Verbindungen wie Phospholipide, beispielsweise Lecitin und Kephaline; Parfümöle, Dimethylisosorbid und Cyclodextrine; faserstrukturverbessernde Wirkstoffe, insbesondere Mono-, Di- und Oligosaccharide wie beispielsweise Glucose, Galactose, Fructose, Fruchtzucker und Lactose; Farbstoffe zum Anfärben des Mittels; Antischuppenwirkstoffe wie Piroctone Olamine, Zink Omadine und Climbazol; Aminosäuren und Oligopeptide; Proteinhydrolysate auf tierischer und/oder pflanzlicher Basis, sowie in Form ihrer Fettsäure-Kondensationsprodukte oder gegebenenfalls anionisch oder kationisch modifizierten Derivate; pflanzliche Öle; Lichtschutzmittel und UV-Blocker; Wirkstoffe wie Panthenol, Pantothensäure, Pantolacton, Allantoin, Pyrrolidinoncarbonsäuren und deren Salze sowie Bisabolol; Polyphenole, insbesondere Hydroxyzimtsäuren, 6,7-Dihydroxycumarine, Hydroxybenzoesäuren, Catechine, Tannine, Leukoanthocyanidine, Anthocyanidine, Flavanone, Flavone und Flavonole; Ceramide oder Pseudoceramide; Vitamine, Provitamine und Vitaminvorstufen; Pflanzenextrakte; Fette und Wachse wie Fettalkohole, Bienenwachs, Montanwachs und Paraffine; Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate; Trübungsmittel wie Latex, Styrol/PVP- und Styrol/Acrylamid-Copolymere; Perlglanzmittel wie Ethylenglykolmono- und - distearat sowie PEG-3-distearat; Pigmente sowie Treibmittel wie Propan-Butan-Gemische, N₂O, Dimethylether, CO₂ und Luft.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher verwiesen. Die zusätzlichen Wirk- und Hilfsstoffe werden in den erfindungsgemäßen Mitteln bevorzugt in Mengen von jeweils 0,0001 bis 25 Gew.-%, insbesondere von 0,0005 bis 15 Gew.-%, jeweils bezogen auf das Gesamtgewicht der Komponente (K1) und/oder der Oxidationsmittelzubereitung (K2), eingesetzt.

Wie bereits zuvor beschrieben besitzt das erfindungsgemäße Verfahren eine sehr gute Eignung zur Aufhellung, Blondierung und Bleiche von dunklen Haaren, wobei eine starke Aufhellung bei gleichzeitiger Minimierung der Verrötung beobachtet werden könnte.

Die in diesem Verfahren eingesetzte spezielle Kombination aus den drei Peroxodisulfaten Kaliumeroxodisulfat, Ammoniumperoxodisulfat und Natriumperoxodisulfat ist in der Lage, gerade die im Haar enthaltenen Phäomelanin-Pigmente effektiv oxidativ zu zerstören. Aus diesem Grund ist das erfindungsgemäße Verfahren auch ganz besonders gut zur Blondierung von Haaren von roter und rötlich-blonder Ausgangshaarfarbe geeignet und führt insbesondere auch bei dieser Ausgangshaarfarbe zu gleichmäßigen, starken Blondierungen mit attraktivem Farbergebnis und minimiertem Rotanteil.

In einer weiteren explizit ganz besonders bevorzugten Ausführungsform ist ein erfindungsgemäßes Verfahren daher dadurch gekennzeichnet, dass es ein Verfahren zur Aufhellung von roten oder blond-roten Haaren ist.

In einer weiteren explizit ganz besonders bevorzugten Ausführungsform ist ein erfindungsgemäßes Verfahren daher dadurch gekennzeichnet, dass die
(ii) Verteilung des anwendungsbereiten Mittesl auf Haaren mit hohem Phäomelanin-Anteil erfolgt.

Unter Haaren mit hohem Phäomelanin-Anteil werden Haare mit einer roten bis blond-roten Ausgangshaarfarbe verstanden.

In einer weiteren explizit ganz besonders bevorzugten Ausführungsform ist ein erfindungsgemäßes Verfahren daher dadurch gekennzeichnet, dass die (ii) Verteilung des anwendungsbereiten Mittels auf Haaren mit einer roten bis blond-roten Ausgangshaarfarbe erfolgt.

Die bevorzugten Ausführungsformen des ersten Erfindungsgegenstands gelten mutatis mutandis auch für den zweiten Erfindungsgegenstand.

### Beispiele

Es wurden die folgenden Formulierungen hergestellt (alle Angaben in Gew.-%)

| Komponente (K1) | (E) erfindungsgemäß | (V) Vergleich |
|---|---|---|
| Britesil C 265 | 36,0 | 36,0 |
| Magnesiumcarbonat | 9,65 | 9,65 |
| Natriumhexametaphosphat | 0,20 | 0,20 |
| Rohagit S hv | 1,00 | 1,00 |
| EDTA | 2,00 | 2,00 |
| Kieselsäure | 0,4 | 0,4 |
| Glycin | 0,6 | 0,6 |
| Cekol 50000 | 2,0 | 2,0 |
| Kaliumpersulfat | 17,00 | 32,00 |
| Ammoniumpersulfat | 15,00 | 10,00 |
| Natriumpersulfat | 10,00 | --- |
| Ariabel Blue 300302 | 0,15 | 0,15 |
| Dimethicon, Dimethiconol | 1,50 | 1,50 |
| Paraffinum Liquidum | ad 100 | ad 100 |

| Komponente (K2) | Oxidationsmittelzubereitung (OX) |
|---|---|
| Emulgade F | 4,0 |
| Kaliumhydroxid | 0,1 |
| Natriumbenzoat | 0,1 |
| Dinatrium Pyrophosphat | 0,1 |
| Dipicolinsäure | 0,1 |
| Etidronsäure (60 %ige wässrige Lösung) | 0,3 |
| Paraffinum Liquidum | 17,0 |
| Wasserstoffperoxid (50 %ige wässrige Lösung) | 18,2 |
| Wasser | ad 100 |

**Eingesetzte Rohstoffe:**

| | |
|---|---|
| Britesil C 265: | Natriumsilikat, Anteil SiO₂: 57,0 - 59,9 Gew.-%, Anteil Na₂O: 21,3 - 22,8 Gew.-%, Mol-Verhältnis SiO₂/Na₂O = 2,65 |
| Rohagit S hv: | Methyl Methacrylat, Methacrylsäure Copolymer (Evonik) |
| Cekol 50000: | Carboxymethylcellulose, Natriumsalz (Cellulose Gum) (CP Kelco, Nordmann Rassmann) |
| Ariabel Blue300302: | CI 77007 (ULTRAMARINES) |
| Emulgade F: | Cetearylalkohol, PEG-40 Castor Oil, Natriumcetearylsulfat (BASF) |

Die Formulierungen (E) und (OX) wurden im Gewichtsverhältnis 1:2 (1 Gewichtsanteil Formulierung (E) und 2 Gewichtsanteile Formulierung (OX)) miteinander vermischt.

Die Formulierungen (V) und (OX) wurden im Gewichtsverhältnis 1:2 (1 Gewichtsanteil Formulierung (V) und 2 Gewichtsanteile Formulierung (OX)) miteinander vermischt.

Vor der Blondierung wurden Haarsträhnen farbmetrisch vermessen. Dann wurden beide anwendungsbereiten Aufhellmittel (E+OX sowie V+OX) jeweils auf Haarsträhnen appliziert und dort für 45 Minuten bei 35°C im Trockenschrank belassen. Danach wurden die Haarsträhnen zunächst mit einem Shampoo, dann mit Wasser gespült und anschließend getrocknet. Im Anschluss an die Trocknung wurden die Strähnen erneut farbmetrisch vermessen.

Aus den erhaltenen Lab-Werten wurde der Farbabstand (ΔE-Wert) zwischen den unbehandelten und den blondierten Haaren ermittelt.

**Farbmetrik-Werte**

| | L | a | b | ΔE |
|---|---|---|---|---|
| unbehandelt | 18,14 | 1,87 | 1,85 | -- |
| blondiert mit V + OX | 50,09 | 12,03 | 32,96 | 45,74 |
| blondiert mit E + OX | 53,01 | 11,44 | 33,12 | 47,81 |

Je höher der L-Wert ist, desto besser ist das Aufhellergebnis.

Je geringer der a-Wert ist, desto geringer ist die Verrötung der Haarsträhne.

## Patentansprüche

1. Kosmetisches Mittel zum Aufhellen keratinischer Fasern, enthaltend - bezogen auf sein Gesamtgewicht
(A) 12,0 bis 23,0 Gew.-% Kaliumperoxodisulfat und
(B) 10,0 bis 20,0 Gew.-% Ammoniumperoxodisulfat und
(C) 5,0 bis 15,0 Gew.-% Natriumperoxodisulfat,
**dadurch gekennzeichnet, dass**
- das Gewichtsverhältnis von (A) zu (B), d.h. das Gewichtsverhältnis (A)/(B), bei einem Wert von 1,0 bis 1,5 liegt,
- das Gewichtsverhältnis von (A) zu (C), d.h. das Gewichtsverhältnis (A)/(C), bei einem Wert von 1,3 bis 2,1 liegt, und
- das Gewichtsverhältnis von (B) zu (C), d.h. das Gewichtsverhältnis (B)/(C), bei einem Wert von 1,1 bis 1,9 liegt.

2. Mittel nach Anspruch 1, **dadurch gekennzeichnet, dass** das Gewichtsverhältnis von (A) zu (B), d.h. das Gewichtsverhältnis (A)/(B), bei einem Wert von 1,0 bis 1,4, weiter bevorzugt von 1,0 bis 1,3 und besonders bevorzugt von 1,0 bis 1,2 liegt.

3. Mittel nach einem der Ansprüche 1 bis 2, **dadurch gekennzeichnet, dass** das Gewichtsverhältnis von (A) zu (C), d.h. das Gewichtsverhältnis (A)/(C), bei einem Wert von 1,4 bis 2,0, weiter bevorzugt von 1,5 bis 1,9 und besonders bevorzugt von 1,6 bis 1,8 liegt.

4. Mittel nach einem der Ansprüche 1 bis 3, **dadurch gekennzeichnet, dass** das Gewichtsverhältnis von (B) zu (C), d.h. das Gewichtsverhältnis (B)/(C), bei einem Wert von 1,2 bis 1,8, weiter bevorzugt von 1,3 bis 1,7 und besonders bevorzugt von 1,4 bis 1,6 liegt.

5. Mittel nach einem der Ansprüche 1 bis 4, **dadurch gekennzeichnet, dass** der Gesamtgehalt aller im Mittel enthaltenen Peroxodisulfate (A) + (B) + (C) - bezogen auf das Gesamtgewicht des Mittels - bei einem Wert von 36,0 bis 48,0 Gew.-%, bevorzugt von 37,0 bis 47,0 Gew.-%, weiter bevorzugt von 38,0 bis 46,0 Gew.-% und besonders bevorzugt von 39,0 bis 45,0 Gew.-% liegt.

6. Mittel nach einem der Ansprüche 1 bis 5, **dadurch gekennzeichnet, dass** es - bezogen auf sein Gesamtgewicht - (A) 13,0 bis 22,0 Gew.-%, bevorzugt 14,0 bis 21,0 Gew.-%, weiter bevorzugt 15,0 bis 20,0 Gew.-% und besonders bevorzugt 16,0 bis 19,0 Gew.-% Kaliumperoxodisulfat enthält.

7. Mittel nach einem der Ansprüche 1 bis 6, **dadurch gekennzeichnet, dass** es - bezogen auf sein Gesamtgewicht - (B) 11,0 bis 19,0 Gew.-%, bevorzugt 12,0 bis 18,0 Gew.-%, weiter bevorzugt 13,0 bis 17,0 Gew.-% und besonders bevorzugt 14,0 bis 16,0 Gew.-% Ammoniumperoxodisulfat enthält.

8. Mittel nach einem der Ansprüche 1 bis 7, **dadurch gekennzeichnet, dass** es - bezogen auf sein Gesamtgewicht - (C) 6,0 bis14,0 Gew.-%, bevorzugt 7,0 bis 13,0 Gew.-%, weiter bevorzugt 8,0 bis 12,0 Gew.-% und besonders bevorzugt 9,0 bis 11,0 Gew.-% Natriumperoxodisulfat enthält.

9. Mittel nach einem der Ansprüche 1 bis 8, **dadurch gekennzeichnet, dass** es ein oder mehrere Alkalisierungsmittel aus der Gruppe aus Magnesiumcarbonat, Magnesiumhydrogencarbonat, Magnesiumhydroxid, Calciumcarbonat, Calciumhydrogencarbonat, Calciumhydroxid, Ammoniumcarbonat, Natriumcarbonat, Natriumhydrogencarbonat, Kaliumcarbonat und Kaliumhydrogencarbonat enthält.

10. Mittel nach einem der Ansprüche 1 bis 9, **dadurch gekennzeichnet, dass** es - bezogen auf sein Gesamtgewicht - 5,0 bis 20,0 Gew.-%, bevorzugt 6,0 bis 17,5 Gew.-%, weiter bevorzugt 7,0 bis 15,0 Gew.-% und besonders bevorzugt 9,0 bis 12,5 Gew.-% Magnesiumcarbonat enthält.

11. Verfahren zur Aufhellung keratinischer Fasern, umfassend die folgenden Schritte in der angegebenen Reihenfolge
(i) Herstellung eines anwendungsbereiten Mittels zur Aufhellung keratinischer Fasern durch Vermischen einer ersten Komponente (K1) mit einer zweiten Komponente (K2),
(ii) Verteilung des anwendungsbereiten Mittels auf den keratinischen Fasern,
(iii) Verbleib des Mittels für einen Zeitraum von 1 bis 60 Minuten auf den Fasern und
(iv) Auswaschen des Mittels aus den Fasern,
wobei
- die erste Komponente (K1) ein Mittel nach einem der Ansprüche 1 bis 10 ist
- die zweite Komponente (K2) eine Oxidationsmittelzubereitung ist, die Wasserstoffperoxid enthält.

12. Verfahren nach Anspruch 11, **dadurch gekennzeichnet, dass** die erste Komponente (K1) und die zweite Komponente (K2) in einem Gewichtsverhältnis (K1)/(K2) von 0,3 bis 1,0, bevorzugt von 0,3 bis 0,9, weiter bevorzugt von 0,3 bis 0,8 und besonders bevorzugt von 0,3 bis 0,7 miteinander vermischt werden.

13. Verfahren nach einem der Ansprüche 11 bis 12, **dadurch gekennzeichnet, dass** die Komponente (K2) - bezogen auf das Gesamtgewicht der Komponente (K2) - 1,5 bis 12,0 Gew.-%, bevorzugt 3,0 bis 11,0 Gew.-%, weiter bevorzugt 4,5 bis 10,0 Gew.-% und besonders bevorzugt 5,5 bis 9,5 Gew.-% Wasserstoffperoxid enthält.

14. Verfahren nach einem der Ansprüche 11 bis 13, **dadurch gekennzeichnet, dass** es ein Verfahren zur Aufhellung von roten oder blond-roten Haaren ist.

15. Verfahren nach einem der Ansprüche 11 bis 14, **dadurch gekennzeichnet, dass** die (ii) Verteilung des anwendungsbereiten Mittels auf Haaren mit hohem Phäomelanin-Anteil erfolgt.

## Claims

1. A cosmetic agent for lightening keratin fibers, containing, based on its total weight,
(A) from 12.0 to 23.0 wt.% of potassium peroxodisulfate and
(B) from 10.0 to 20.0 wt.% of ammonium peroxodisulfate and
(C) from 5.0 to 15.0 wt.% of sodium peroxodisulfate,
**characterized in that**
- the weight ratio of (A) to (B), i.e. the weight ratio (A)/(B), is from 1.0 to 1.5,
- the weight ratio of (A) to (C), i.e. the weight ratio (A)/(C), is from 1.3 to 2.1, and
- the weight ratio of (B) to (C), i.e. the weight ratio (B)/(C), is from 1.1 to 1.9.

2. The agent according to claim 1, **characterized in that** the weight ratio of (A) to (B), i.e. the weight ratio (A)/(B), is from 1.0 to 1.4, more preferably from 1.0 to 1.3, and particularly preferably from 1.0 to 1.2.

3. The agent according to one of claims 1 to 2, **characterized in that** the weight ratio of (A) to (C), i.e. the weight ratio (A)/(C), is from 1.4 to 2.0, more preferably from 1.5 to 1.9, and particularly preferably from 1.6 to 1.8.

4. The agent according to one of claims 1 to 3, **characterized in that** the weight ratio of (B) to (C), i.e. the weight ratio (B)/(C), is from 1.2 to 1.8, more preferably from 1.3 to 1.7, and particularly preferably from 1.4 to 1.6.

5. The agent according to one of claims 1 to 4, **characterized in that** the total content of all peroxodisulfates (A) + (B) + (C) contained in the agent, based on the total weight of the agent, is from 36.0 to 48.0 wt.%, preferably from 37.0 to 47.0 wt.%, more preferably from 38.0 to 46.0 wt.%, and particularly preferably from 39.0 to 45.0 wt.%.

6. The agent according to one of claims 1 to 5, **characterized in that** it contains, based on its total weight, (A) from 13.0 to 22.0 wt.%, preferably from 14.0 to 21.0 wt.%, more preferably from 15.0 to 20.0 wt.%, and particularly preferably from 16.0 to 19.0 wt.%, of potassium peroxodisulfate.

7. The agent according to one of claims 1 to 6, **characterized in that** it contains, based on its total weight, (B) from 11.0 to 19.0 wt.%, preferably from 12.0 to 18.0 wt.%, more preferably from 13.0 to 17.0 wt.%, and particularly preferably from 14.0 to 16.0 wt.%, of ammonium peroxodisulfate.

8. The agent according to one of claims 1 to 7, **characterized in that** it contains, based on its total weight, (C) from 6.0 to 14.0 wt.%, preferably from 7.0 to 13.0 wt.%, more preferably from 8.0 to 12.0 wt.%, and particularly preferably from 9.0 to 11.0 wt.%, of sodium peroxodisulfate.

9. The agent according to one of claims 1 to 8, **characterized in that** it contains one or more alkalizing agents from the group of magnesium carbonate, magnesium hydrogen carbonate, magnesium hydroxide, calcium carbonate, calcium hydrogen carbonate, calcium hydroxide, ammonium carbonate, sodium carbonate, sodium hydrogen carbonate, potassium carbonate and potassium hydrogen carbonate.

10. The agent according to one of claims 1 to 9, **characterized in that** it contains, based on its total weight, from 5.0 to 20.0 wt.%, preferably from 6.0 to 17.5 wt.%, more preferably from 7.0 to 15.0 wt.%, and particularly preferably from 9.0 to 12.5 wt.%, of magnesium carbonate.

11. A method for lightening keratin fibers, comprising the following steps in the stated sequence
(i) preparing a ready-to-apply agent for lightening keratin fibers by mixing a first component (K1) with a second component (K2),
(ii) distributing the ready-to-apply agent onto the keratin fibers,
(iii) leaving the agent on the fibers for a period of 1 to 60 minutes and
(iv) washing the agent out of the fibers,
wherein
- the first component (K1) is an agent according to one of claims 1 to 10
- the second component (K2) is an oxidizing agent preparation containing hydrogen peroxide.

12. The method according to claim 11, **characterized in that** the first component (K1) and the second component (K2) are mixed with one another in a weight ratio (K1)/(K2) of from 0.3 to 1.0, preferably from 0.3 to 0.9, more preferably from 0.3 to 0.8, and particularly preferably from 0.3 to 0.7.

13. The method according to one of claims 11 to 12, **characterized in that** the component (K2) contains, based on the total weight of the component (K2), from 1.5 to 12.0 wt.%, preferably from 3.0 to 11.0 wt.%, more preferably from 4.5 to 10.0 wt.%, and particularly preferably from 5.5 to 9.5 wt.%, of hydrogen peroxide.

14. The method according to one of claims 11 to 13, **characterized in that** it is a method for lightening red or red-blonde hair.

15. The method according to one of claims 11 to 14, **characterized in that** the
(ii) ready-to-apply agent is distributed onto hair that has a high pheomelanin proportion.

## Revendications

1. Agent cosmétique pour l'éclaircissement de fibres kératiniques, contenant, par rapport à son poids total,
(A) 12,0 à 23,0 % en poids de peroxodisulfate de potassium ;
(B) 10,0 à 20,0 % en poids de peroxodisulfate d'ammonium ; et
(C) 5,0 à 15,0 % en poids de peroxodisulfate de sodium,
**caractérisé en ce que**
- le rapport pondéral de (A) à (B), c'est-à-dire le rapport pondéral (A)/(B), a une valeur située dans la plage allant de 1,0 à 1,5,
- le rapport pondéral de (A) à (C), c'est-à-dire le rapport pondéral (A)/(C), a une valeur située dans la plage allant de 1,3 à 2,1, et
- le rapport pondéral de (B) à (C), c'est-à-dire le rapport pondéral (B)/(C), a une valeur située dans la plage allant de 1,1 à 1,9.

2. Agent selon la revendication 1, **caractérisé en ce que** le rapport pondéral de (A) à (B), c'est-à-dire le rapport pondéral (A)/(B), a une valeur située dans la plage allant de 1,0 à 1,4, de manière davantage préférée de 1,0 à 1,3 et de manière particulièrement préférée de 1,0 à 1,2.

3. Agent selon l'une des revendications 1 à 2, **caractérisé en ce que** le rapport pondéral de (A) à (C), c'est-à-dire le rapport pondéral (A)/(C), a une valeur située dans la plage allant de 1,4 à 2,0, de manière davantage préférée de 1,5 à 1,9 et de manière particulièrement préférée de 1,6 à 1,8.

4. Agent selon l'une des revendications 1 à 3, **caractérisé en ce que** le rapport pondéral de (B) à (C), c'est-à-dire le rapport pondéral (B)/(C), a une valeur située dans la plage allant de 1,2 à 1,8, de manière davantage préférée de 1,3 à 1,7 et de manière particulièrement préférée de 1,4 à 1,6.

5. Agent selon l'une des revendications 1 à 4, **caractérisé en ce que** la teneur totale de tous les peroxodisulfates (A) + (B) + (C) contenus dans l'agent, par rapport au poids total de l'agent, a une valeur située dans la plage allant de 36,0 à 48,0 % en poids, de préférence de 37,0 à 47,0 % en poids, de manière davantage préférée de 38,0 à 46,0 % en poids et de manière particulièrement préférée de 39,0 à 45,0 % en poids.

6. Agent selon l'une des revendications 1 à 5, **caractérisé en ce qu'**il contient, par rapport à son poids total, (A) de 13,0 à 22,0 % en poids, de préférence de 14,0 à 21,0 % en poids, de manière davantage préférée de 15,0 à 20,0 % en poids et de manière particulièrement préférée de 16,0 à 19,0 % en poids de peroxodisulfate de potassium.

7. Agent selon l'une des revendications 1 à 6, **caractérisé en ce qu'**il contient, par rapport à son poids total, (B) de 11,0 à 19,0 % en poids, de préférence de 12,0 à 18,0 % en poids, de manière davantage préférée de 13,0 à 17,0 % en poids et de manière particulièrement préférée de 14,0 à 16,0 % en poids de peroxodisulfate d'ammonium.

8. Agent selon l'une des revendications 1 à 7, **caractérisé en ce qu'**il contient, par rapport à son poids total, (C) de 6,0 à 14,0 % en poids, de préférence de 7,0 à 13,0 % en poids, de manière davantage préférée de 8,0 à 12,0 % en poids et de manière particulièrement préférée de 9,0 à 11,0 % en poids de peroxodisulfate de sodium.

9. Agent selon l'une des revendications 1 à 8, **caractérisé en ce qu'**il contient un ou plusieurs agents d'alcalinisation choisis dans le groupe comprenant le carbonate de magnésium, l'hydrogénocarbonate de magnésium, l'hydroxyde de magnésium, le carbonate de calcium, l'hydrogénocarbonate de calcium, hydroxyde de calcium, le carbonate d'ammonium, le carbonate de sodium, l'hydrogénocarbonate de sodium, le carbonate de potassium et l'hydrogénocarbonate de potassium.

10. Agent selon l'une des revendications 1 à 9, **caractérisé en ce qu'**il contient, par rapport à son poids total, 5,0 à 20,0 % en poids, de préférence 6,0 à 17,5 % en poids, de manière davantage préférée 7,0 à 15,0 % en poids et de manière particulièrement préférée 9,0 à 12,5 % en poids de peroxodisulfate de magnésium.

11. Procédé d'éclaircissement de fibres kératiniques, comprenant les étapes suivantes, dans l'ordre indiqué :
i) Préparation d'un agent prêt à l'emploi pour l'éclaircissement des fibres kératiniques en mélangeant un premier composant (K1) avec un second composant (K2),
ii) Répartition de l'agent prêt à l'emploi sur les fibres kératiniques,
iii) Maintien de l'agent sur les fibres pendant une durée de 1 à 60 minutes, et
iv) Lavage de l'agent pour l'enlever des fibres,
dans lequel
- le premier composant (K1) est un agent selon l'une des revendications 1 à 10
- le second composant (K2) est une préparation d'agent oxydant contenant du peroxyde d'hydrogène.

12. Procédé selon la revendication 11, **caractérisé en ce que** le premier composant (K1) et le second composant (K2) sont mélangés dans un rapport pondéral (K1)/(K2) de 0,3 à 1,0, de préférence de 0,3 à 0,9, de manière davantage préférée de 0,3 à 0,8 et de manière particulièrement préférée de 0,3 à 0,7.

13. Procédé selon l'une des revendications 11 à 12, **caractérisé en ce que** le composant (K2) contient, par rapport à son poids total du composant (K2), 1,5 à 12,0 % en poids, de préférence 3,0 à 11,0 % en poids, de manière davantage préférée 4,5 à 10,0 % en poids et de manière particulièrement préférée 5,5 à 9,5 % en poids de peroxyde d'hydrogène.

14. Procédé selon l'une des revendications 11 à 13, **caractérisé en ce qu'**il s'agit d'un procédé d'éclaircissement de cheveux roux ou blonds roux.

15. Procédé selon l'une des revendications 11 à 14, **caractérisé en ce que** la
ii) répartition de l'agent prêt à l'emploi s'effectue sur des cheveux avec une forte proportion de phéomélanine.
